# EUROPEAN PATENT SPECIFICATION

(11) **EP 4 201 895 B1**
(45) Date of publication and mention of the grant of the patent: **06.08.2025**
(21) Application number: 22209187.8
(22) Date of filing: 23.11.2022
(51) Int. Cl.: A61B 90/80, A61G 7/00, A61L 2/00, C02F 103/02, E03C 1/32, A47K 1/02, C02F 1/78

(54) **PORTABLE DISINFECTANT WASHBASIN**
TRAGBARES DESINFEKTIONSWASCHBECKEN
LAVABO POUR DESINFECTANT PORTATIF

(30) Priority: 21.12.2021 IT 202100032060
(43) Date of publication of application: 28.06.2023
(73) Proprietor: Rocco Lagano S.r.l., 26900 Lodi (LO) (IT)
(72) Inventor: Lagano, Rocco, 26900 Lodi (IT)
(74) Representative: Garavelli, Paolo

(56) References cited:
- WO-A1-97/49636
- CA-A1- 2 437 426
- KR-A- 20200 028 633
- US-A1- 2020 407 666
- US-A1- 2021 378 454

## Description

The present invention refers to a portable disinfectant basin for preventing the spread of contagious pathologies and/or for disinfecting the skin, disinfecting and treating compression ulcers, surgical wounds and skin lesions.

The challenge that awaits the scientific community in the coming years is as complex as it is important; it must find solutions to the many problems that are causing thousands of deaths and permanent disabilities all over the world:
- antibiotic resistance;
- Healthcare Associated Infections (HAI);
- the prevention of contagious diseases through the use of a universal disinfection method called "ozonredy";
- role of the universal disinfection system called "ozonredy care" in the disinfection and treatment of compression ulcers, surgical wounds and skin lesions.

The term antibiotic resistance refers to the ability of a bacterium to resist an antibiotic drug.

This type of resistance can be both natural (when the bacterium is naturally resistant to an antibiotic) and acquired (when a bacterium adapts to resist an antibiotic drug through modifications to its genetic heritage).

Antibiotics are a very important resource for health since, starting from their discovery, they have made a decisive contribution to preventing the spread of bacterial infections, reducing their serious complications to a minimum.

Many of the diseases that were not curable in an era before the use of antibiotics are easily curable today.

In recent years, however, antibiotics that were commonly used to treat bacterial infections (such as penicillin in pneumonia) have become less effective or no longer work due to their overuse or misuse.

The phenomenon has reached such proportions as to induce various international institutions to sound the alarm: in Europe about 33,000 people die every year due to infections with antibiotic-resistant bacteria.

The fact that bacteria develop resistance to an antibiotic is a natural evolutionary process but this phenomenon is accelerated and aggravated by the excessive and often incorrect use of these drugs.

Any bacterium that survives an antibiotic treatment can become resistant to subsequent treatments, multiply and transfer its ability to resist antibiotics to other bacteria.

It should be emphasized that the risk of being infected with antibiotic-resistant bacteria concerns not only the person who takes antibiotics improperly but also those who will subsequently be infected by those same bacteria.

A further problem is given by bacteria that become resistant to several antibiotics at the same time (multi-resistance) since in these cases finding a cure becomes very difficult.

In fact, the decrease in the effectiveness of existing antibiotics is not compensated by the discovery of new molecules, and, with the increase in resistance, it will become increasingly difficult to recover from infections.

Among the most important bacterial species that have become resistant to antibiotics are *staphylococcus aureus,* which can cause infections of the skin and of the whole body (septicaemia); *klebsiella pneumoniae,* which causes septicaemia, urinary and pulmonary infections; campylobacter, which causes intestinal infections, and *Escherichia coli* which can cause different types of infections, among which the most common are urinary tract infections.

Infections caused by antibiotic-resistant bacteria can be very serious and difficult to treat and are becoming a major public health problem worldwide.

The spread of resistant bacteria in hospitals or healthcare facilities is a major problem for people's safety as it increases the number of cases of illness and death, as well as the length of hospital stays.

In Italy about 30-60% of the bacteria that cause hospital infections are resistant to the most commonly used antibiotics (first choice).

The major concern is that bacteria resistant to all available antibiotics (pan-resistant) may develop.

Cases of almost total resistance have already been reported in our country.

If antibiotics become ineffective in the future and what is referred to as the post-antibiotic era sets in, even common infections and light wounds that have been easily healed for decades could once again be a threat to health. Furthermore, it would become risky to perform surgical operations or perform transplants, implantation of prostheses or chemotherapy treatments.

Italy is the European nation in which the mortality caused by antibiotic resistance is highest: out of about 33,000 deaths a year in all of Europe, there are over 10,000 in our country.

In 2050, according to recent studies, infections with antibiotic resistant bacteria will kill more than cancer.

Any type of infection, from as simple as a simple skin or urinary tract infection, to serious infections such as pneumonia and sepsis can be caused by antibiotic-resistant bacteria.

It seems a paradox, but even a person who has never taken antibiotics runs the risk of having an infection with resistant bacteria.

In EU countries there were 671,689 cases of antibiotic-resistant infections to which 33,110 deaths are attributable, especially in children in the first months of life and in the elderly.

Of these infections, 63% are health and social care-associated infections (HAI).

Healthcare-related infections (HAI) are infections acquired in all areas of care, including acute care hospitals, day-hospital/day-surgery, long-term care, outpatient clinics, home care, community residential facilities.

In fact, they are the most frequent and serious complication of health care.

HAIs are the effect of the progressive introduction of new health technologies, which, if on the one hand improve the therapeutic possibilities and the outcome of the disease, on the other hand can allow the entry of microorganisms even in normally sterile body sites, causing diseases severe or even death.

The emergence of bacterial strains resistant to antibiotics, given the widespread use of these drugs for prophylactic or therapeutic purposes, further complicates the course of many HAIs, since in fact the circulation of these pathogens is greater in the healthcare setting and, due to the greater difficulties of treatment, cause a further increase in the clinical impact.

These infections have a significant clinical and economic impact: according to the first global report of the World Health Organization, HAIs cause a prolongation of hospitalization, long-term disability, increased resistance of microorganisms to antibiotics, an additional economic burden for health systems and for patients and their families and significant excess mortality.

In Europe, HAIs result in 16 million additional days of hospitalization, 37,000 attributable deaths, and 110,000 deaths for which infection is a contributing cause each year. Costs are estimated at approximately 7 billion Euros, including direct costs only.

HAIs are a frequent phenomenon, a recent national prevalence study, conducted using the ECDC protocol, found a frequency of patients with an infection contracted during hospitalization equal to 6.3 for every 100 patients present in hospital; in home care 1 patient out of 100 contracts a HAI.

Not all HAIs are preventable, but it is currently estimated that more than 50% are preventable.

Most of the HAIs affect the urinary tract, the respiratory system, surgical wounds, systemic infections (sepsis, bacteraemia).

The most frequent are urinary infections, which alone account for 35-40% of all hospital infections.

The microorganisms involved vary over time. Until the early 1980s, HAIs were mainly due to gram-negative bacteria (for example, *E. coli* and *Klebsiella pneumoniae) .*

Then, due to the effect of the antibiotic pressure and the greater use of plastic sanitary devices, the infections caused by gram-positives (above all *Enterococci* and *Staphylococcus epidermidis)* and those caused by fungi (above all Candida) have increased, while those caused by gram-negatives have decreased.

However, recently, some gram-negatives, such as carbapenemase-producing enterobacteria and *Acinetobacter spp.,* responsible for serious infections, have become very frequent in the hospital care setting.

The people at greatest risk of contracting a HAI are the clients; however, they are exposed and staff and visitors can also be affected.

Among the conditions that increase the susceptibility to infections are age (infants and the elderly), other infections or serious concomitant pathologies (tumours, immunodeficiency, diabetes, anaemia, heart disease, renal insufficiency, transplants), malnutrition, trauma, burns, exposure to particular invasive care techniques (catheters, surgery, endoscopy).

Like other infections, HAIs can be transmitted by direct person-to-person contact (especially through the hands, droplets emitted during speech, sneezing or coughing, or by air) or indirectly through contaminated objects (both diagnostic tools or welfare, and common objects); the modality changes according to the pathogen.

The prevention and control of HAI therefore represent essential interventions to reduce the impact of these infections and more generally to reduce the spread of antibiotic-resistant microorganisms.

One of the crucial points for combating HAI is the definition and application of good care practices and other measures, according to an integrated program that must be adapted to each care setting.

Among the key measures, we mention the correct washing of hands (which remains one of the most important and effective), the reduction of unnecessary diagnostic and therapeutic procedures, the correct use of antibiotics and disinfectants, the sterilization of devices, the respect of asepsis in invasive procedures, control of the risk of environmental infection, protection of patients with appropriate use of antibiotic prophylaxis and administration of recommended vaccinations (when possible with adequate advance to allow for an adequate immune response), infection surveillance, identification and control of epidemics, vaccination of health workers.

Prevention in the healthcare setting requires constantly correct behaviour on the part of health professionals and anyone who frequents these environments.

Lack of knowledge of the rules or inattention can lead to incorrect behaviour, a potential cause of the transmission of pathogenic bacteria and consequent HAI.

In the case of HAI, adequate control interventions are also important, such as the immediate identification of cases and the mode of transmission - through epidemiological investigations, their adequate treatment, possible isolation from other patients, the reinforcement of the measures that are already standard must be adopted to avoid transmission between patients.

Documents US-A1-2021/378454, CA-A1-2 437 426, KR-A-2020 0028633, US-A1-2020/407666 and WO-A1-97/49636 disclose washbasins and disinfecting solutions according to the prior art.

In particular, document US 2021/378454 A1 discloses a portable disinfectant washbasin comprising a containing element with a bin compartment, said bin compartment being operatively connected to a lifting pump designed to send the water to a tap and which can be operated by means of at least one pedal; the containing element comprises furthermore a tray for collecting said used water, said tray being operatively connected to a discharge/collection bin of said used water; and a soap dish operatively connected to said containing element with automatic dispensing.

Object of the present invention is solving the aforesaid prior art problems by supplying, through a portable disinfectant washbasin equipped with an ozonizer and the collection of used water capable of sanitizing and/or disinfecting the patient's skin, at the same time the healthcare operator using the ozonized water takes advantage of eliminating germs and viruses present on gloves or hands, significantly reducing the possibility of spreading germs and viruses both in the environment and from patient to patient, this method favours the reduction of HAIs with considerable economic advantage for the NHS which in terms of saving lives and disability.

The above and other objects and advantages of the invention, as will appear from the following description, are achieved with a portable disinfectant washbasin such as the one described in claim 1. Preferred embodiments and non-trivial variants of the present invention form the subject of the dependent claims.

It is understood that all attached claims form an integral part of the present description.

It will be immediately obvious that innumerable variations and modifications may be made to what has been described (for example relating to shape, dimensions, arrangements and parts with equivalent functions) without departing from the scope of protection of the invention as appears from the attached claims.

The present invention will be better described by some preferred embodiments, provided by way of non-limiting example, with reference to the attached drawings, in which:
- FIG. 1 shows a view of the inside of an embodiment of the portable disinfectant basin according to the present invention; and

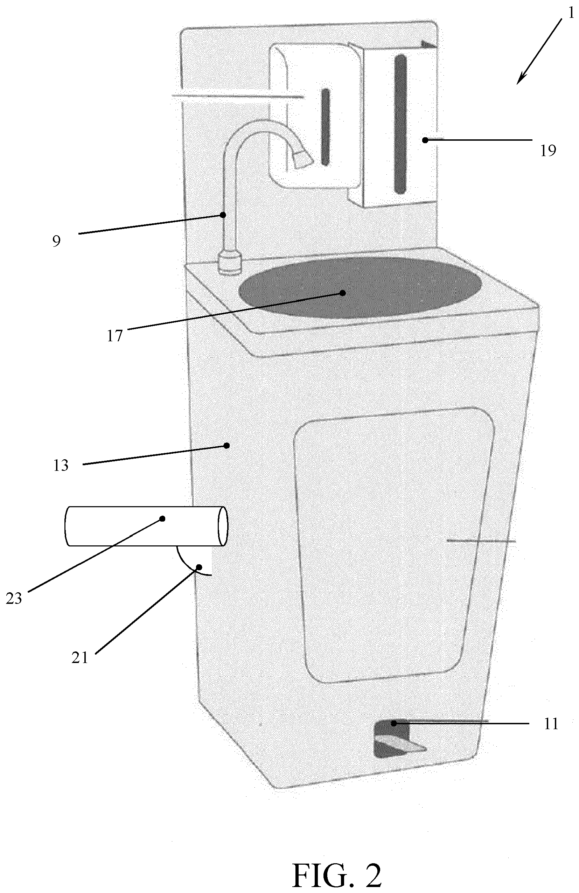

- FIG. 2 shows a perspective view of an embodiment of the portable disinfectant basin according to the present invention.

As a form of prevention of the spread of contagious diseases, a new universal disinfection method called "ozonredy" is proposed.

The natural element used in this sense, as demonstrated by numerous studies, is precious: ozone.

Of all the disinfectants in the world, ozone is the best at killing germs and bacteria.

Its very high oxidizing power makes it an effective sanitizer, even stronger than traditional chemical detergents. It is 100% natural, ecological and economical.

Thanks to its properties, ozone sanitizes, oxygenates and regenerates the air we breathe and disinfects the water we use.

It eliminates over 99.9% of bacteria, moulds, fungi, yeasts, pollen and mites and inactivates viruses. It is a powerful ally in the fight against infections, because it breaks down the microbial load present in the air and on surfaces.

It disinfects perfectly without the need for chemical additives and detergents, exploiting its natural oxidizing power.

Once the treatment is finished, the ozone converts back into oxygen without leaving any toxic or chemical residue.

As stated, ozone is an unstable gas, at 20 °C it has a half-life of three days, in an aqueous solution of 20 minutes.

It cannot therefore be stored, it must be produced at the time of use, which is why "ozonredy" is decisive for the primary prevention of contagious diseases.

We have stated that the use of "ozonredy" is essential to prevent HAIs but do not forget the other beneficial actions that ozone brings.

While proceeding to the personal hygiene of the patient at the same time, with "ozonredy" care, the same compression ulcers and surgical wounds are disinfected, in addition to disinfecting them, it accelerates their healing through a well-known mechanism, i.e. by promoting a greater blood flow to the treated lesions, the same therefore thanks to the better oxygenation and the greater supply of nutrients they halve the healing times with considerable advantages for the patient and for the coffers of the NHS.

"Ozonredy" is effective in deactivating the pathogenic action of bacteria, viruses, fungi, moulds and insects (sources: Edelstein et al.,1982; Joret et al.,1982; Farooq and Akhlaque, 1983; Harakeh and Butle,1986; Kawamuram et al. 1986).

**Table 1**

| ORGANISM | CONCENTRATION |
|---|---|
| BACTERIA | |
| (E. Coli, Legionella, Mycobacterium, Fecal Streptococcus) | 0.23 ppm - 2.2 ppm |
| VIRUS | |
| (Poliovirus type-1, Human Rotavirus, Enteric virus) | 0.2 ppm - 4.1 ppm |
| MOLDS | |
| (Aspergillus Niger, various strains of Penicillum, Cladosporium) | 2 ppm |
| FUNGUS | |
| (Candida Parapsilosis, Candida Tropicalis) | 0.02 ppm - 0.26 ppm |
| INSECTS | |
| (Acarus Siro, Tyrophagus Casei, Tyrophagus Putrescientiae) | 1.5 - 2 ppm |

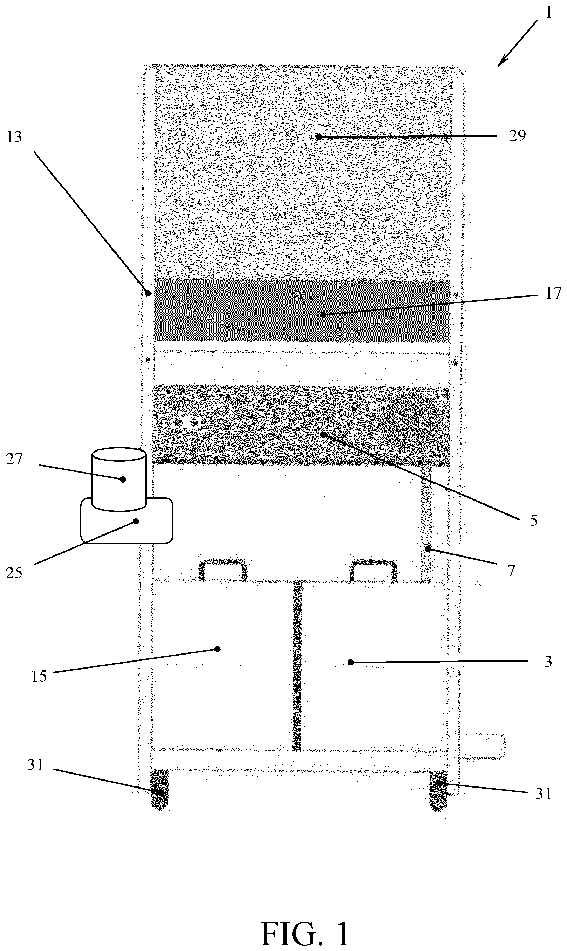

Referring to FIG. 1, it can be seen that the portable disinfectant washbasin 1 comprises at least one containing element 13 comprising at least one can holder compartment 3, with a containing capacity of 20 litres of water, operatively connected to at least one portable ozonator device 5 which is capable of saturating water contained therein in seven/ten minutes.

Advantageously, the bin compartment 3 is also operatively connected to at least one lifting pump 7 designed to send the ozonated water to at least one tap 9 which can be operated by means of at least one pedal 11.

Furthermore, the portable disinfectant washbasin 1 according to the present invention comprises a tray 17 for collecting the ozonized water once it is used for disinfection; the tray 17 is operatively connected to at least one drain/collection bin 15 where the water ozonated gas used is definitively accumulated.

In particular, installed on the containing element 13 there is a soap dish 19 (antibacterial-antiviral) which is designed to dispense ozonized liquid soap with automatic dispensing by means of a photocell.

Advantageously, on one of the sides of the containing element 13, there is at least one hook 21 designed to hang at least one extension tube 23 capable of operatively connecting to a tap (not shown) to allow filling of the bin holder compartment 3 without therefore moving it from its location.

Furthermore, the portable disinfectant washbasin 1 is provided with at least one platform 25 with safety straps operatively connected to the containing element 13.

The platform 25 is designed to fix, on one of the sides of the portable disinfectant basin 1, a cylinder of pure O₂ 27, this cylinder of pure O₂ 27 being connected to the portable ozonator device 5.

In particular, the portable ozonator device 5 can be both electric and battery operated.

Advantageously, the portable disinfectant washbasin 1 can comprise at least one splash guard element 29 operatively connected to the containing element 13 and capable of protecting the user from any ozonated water leaks.

Furthermore, the portable disinfectant washbasin 1 can be equipped with wheels 31 to facilitate its transport or movement from one area to another and, in addition, it can be made of stainless steel or resin.

Technical Sheet of the portable disinfectant washbasin 1:

**Table 2**

| | |
|---|---|
| Total sizes (width x depth x height) | 50cm x 50cm x 125cm |
| Washbasin sizes without splash guard edge | 55cm x 55cm x 90 cm |
| Wheel diameter | 50 mm |
| Weight | 17 / 20 kg |

Ozone is universally recognized as the most effective natural gas for eliminating germs, bacteria, viruses and fungi.

As already mentioned, ozone binds easily to water molecules but the union is unstable due to the volatility of the gas; the bond lasts a few minutes, and therefore it is not possible to store the ozonated water previously prepared for the patient's personal hygiene or for the washing of the hands of healthcare workers, so it must be prepared and used at the moment.

Using "ozonredy" the desired solution is quickly obtained: it is therefore not necessary to resort to antibiotic therapy to treat HAIs as "ozoredy" prevents them; in addition to hygiene and disinfection, the ozonized solution stimulates and promotes blood circulation.

Covid 19 pandemic - this virus is straining all health systems globally as well as claiming tens of thousands of lives.

It took months to figure out how to treat patients with cov-sars-2 and take the right precautionary measures to limit the spread of the virus.

Given the proven ability of ozone to deactivate any bacterial and viral form, "ozonredy" is indispensable, as well as in hospitalization and treatment institutions, even where the coexistence of several people is necessary by necessity, furthermore given the absence of side effects the ozone can be used in nursery schools, early childhood schools, in the intimate hygiene of new mothers and the unborn child, company canteens, medical and veterinary surgeries, family use, etc.

Finally, for optimal performance of the "ozonredy" and "ozonredy care" hygiene/disinfection system, it is recommended to use the antibacterial wipe by DRY&SAFE, owner of EU patent n° EP2228044A2, to dry the treated parts.

## Claims

1. Portable disinfectant washbasin (1) comprising at least one tap(9) and at least one containing element (13), said containing element comprising:
- at least one bin compartment (3) operatively connected to at least one lifting pump (7);
- a portable ozonator device (5) designed to saturate water contained therein;
- a tray (17) for collecting used ozonized water, said tray (17) being operatively connected to at least one discharge/collection bin (15) of said used ozonized water;
- at least one soap dish (19) operatively connected to said containing element (13);
- at least one pedal (11); wherein the containing element (13) further comprises:
- a cylinder of pure 02 (27);
- at least one platform (25) with safety straps operatively connected to said containing element (13) designed to fix the cylinder of pure O₂ (27), said cylinder of pure O₂ (27) being operatively connected to said portable ozonizer device (5);
- the at least one bin compartment (3) is operatively connected to the at least one portable ozonator device (5);
- the lifting pump (7) is designed to send the ozonated water to the at least one tap (9) and is operated by means of the at least one pedal (11); and
- the soap dish (19) dispenses ozonated liquid soap with automatic dispensing by means of a photocell.

2. Portable disinfectant washbasin (1) according to claim 1, **characterized in that** it also comprises at least one hook (21) operatively connected to said containing element (13) designed to hang at least one extension tube (23) designed to operatively connect to a tap to allow filling of the bin compartment (3).

3. Portable disinfectant washbasin (1) according to claim 1 or 2, **characterized in that** said portable ozonator device (5) is electric and/or battery operated.

4. Portable disinfectant washbasin (1) according to claim 1, 2 or 3, **characterized in that** it comprises at least one splash guard element (29) operatively connected to said containing element (13).

5. Portable disinfectant washbasin (1) according to any one of the preceding claims, **characterized in that** it comprises a plurality of wheels (31).

6. Portable disinfectant washbasin (1) according to any one of the preceding claims, **characterized in that** said portable disinfectant washbasin (1) is made of stainless steel or resin.

## Patentansprüche

1. Tragbares Desinfektionsbecken (1) mit:
- mindestens einen Hahn (9) und
- mindestens ein Eindämmungselement (13), wobei das Eindämmungselement umfasst:
- mindestens ein Behälterfach (3), das mit mindestens einer Hebepumpe (7) betriebsmäßig verbunden ist;
- ein tragbares Ozonisierungsgerät (5), das dazu bestimmt ist, das darin enthaltene Wasser zu sättigen;
- einen Sammeltank (17) für ozonisiertes Gebrauchtwasser, wobei der Tank (17) betriebsmäßig mit mindestens einem Ablass-/Sammelbehälter (15) für das ozonisierte Gebrauchtwasser verbunden ist;
- mindestens eine Seifenschale (19), die betriebsmäßig mit dem Behälterelement (13) verbunden ist;
- mindestens ein Pedal (11), bei dem das Aufnahmeelement (13) außerdem umfasst:
- eine Flasche mit reinem O2 (27);
- mindestens eine Plattform (25) mit Sicherheitsgurten, die betriebsmäßig mit dem Einschlusselement (13) verbunden ist und dazu dient, die Flasche (27) mit reinem O₂ zu sichern, wobei die Flasche (27) mit reinem O₂ betriebsmäßig mit dem tragbaren Ozonisierungsgerät (5) verbunden ist;
- das mindestens eine Behälterfach (3) ist betriebsmäßig mit der mindestens einen tragbaren Ozonisierungsvorrichtung (5) verbunden;
- die Hebepumpe (7) ist dazu ausgelegt, ozonisiertes Wasser an mindestens einen Wasserhahn (9) zu fördern und kann über mindestens ein Pedal (11) bedient werden;
und
- Der Seifenhalter (19) gibt ozonisierte Flüssigseife mit automatischer Dosierung über eine Fotozelle ab.

2. Tragbares Desinfektionsbecken (1) nach Anspruch 1, **dadurch gekennzeichnet, dass** es außerdem mindestens einen Haken (21) umfasst, der betriebsmäßig mit dem Behälterelement (13) verbunden ist und dazu bestimmt ist, mindestens ein Verlängerungsrohr (23) aufzuhängen, das betriebsmäßig mit einem Hahn verbunden werden kann, um das Befüllen des Abfallbehälterfachs (3) zu ermöglichen.

3. Tragbares Desinfektionsbecken (1) nach Anspruch 1 oder 2, **dadurch gekennzeichnet, dass** das tragbare Ozonisierungsgerät (5) elektrisch und/oder batteriebetrieben ist.

4. Tragbares Desinfektionsbecken (1) nach Anspruch 1, 2 oder 3, **dadurch gekennzeichnet, dass** es mindestens ein Spritzschutzelement (29) umfasst, das mit dem Eindämmungselement (13) wirkverbunden ist.

5. Tragbares Desinfektionsbecken (1) nach einem der vorhergehenden Ansprüche, **dadurch gekennzeichnet, dass** es mehrere Räder (31) aufweist.

6. Tragbares Desinfektionsbecken (1) nach einem der vorhergehenden Ansprüche, **dadurch gekennzeichnet, dass** das tragbare Desinfektionsbecken (1) aus rostfreiem Stahl oder Harz hergestellt ist.

## Revendications

1. Évier désinfectant portable (1) comprenant:
- au moins un robinet (9) et
- au moins un élément de confinement (13), ledit élément de confinement comprenant:
- au moins un compartiment de bac (3) relié fonctionnellement à au moins une pompe de levage (7) ;
- un dispositif d'ozonisation portable (5) conçu pour saturer l'eau qu'il contient;
- un réservoir de collecte (17) pour l'eau ozonée usagée, ledit réservoir (17) étant relié fonctionnellement à au moins un bac d'évacuation/collecte (15) pour ladite eau ozonée usagée;
- au moins un porte-savon (19) relié fonctionnellement audit élément de confinement (13);
- au moins une pédale (11) dans laquelle l'élément de retenue (13) comprend également:
- une bouteille d'O2 pur (27);
- au moins une plate-forme (25) avec des sangles de sécurité reliées fonctionnellement audit élément de confinement (13) conçue pour fixer la bouteille d'O2 pur (27), ladite bouteille d'O2 pur (27) étant reliée fonctionnellement audit appareil d'ozonisation portable (5);
- le ou les compartiments de bac (3) sont reliés fonctionnellement au ou aux dispositifs d'ozonisation portables (5);
- la pompe de relevage (7) est conçue pour envoyer de l'eau ozonée vers au moins un robinet (9) et peut être actionnée par l'intermédiaire d'au moins une pédale (11) ;
et
- le porte-savon (19) distribue du savon liquide ozoné avec distribution automatique via cellule photoélectrique.

2. Évier désinfectant portable (1) selon la revendication 1, **caractérisé en ce qu'**il comprend également au moins un crochet (21) relié fonctionnellement audit élément de récipient (13) conçu pour suspendre au moins un tube d'extension (23) conçu pour se connecter fonctionnellement à un robinet pour permettre le remplissage du compartiment de bac (3).

3. Évier désinfectant portable (1) selon la revendication 1 ou 2, **caractérisé en ce que** ledit appareil d'ozonisation portable (5) est alimenté par un courant électrique et/ou par batterie.

4. Évier désinfectant portable (1) selon la revendication 1, 2 ou 3, **caractérisé par le fait qu'**il comprend au moins un élément anti-éclaboussures (29) relié fonctionnellement audit élément de confinement (13).

5. Évier désinfectant portable (1) selon l'une quelconque des revendications précédentes, **caractérisé en ce qu'**il comprend une pluralité de roues (31).

6. Évier désinfectant portable (1) selon l'une quelconque des revendications précédentes, **caractérisé en ce que** ledit évier désinfectant portable (1) est réalisé en acier inoxydable ou en résine.
